(19) Europäisches Patentamt
European Patent Office
Office européen des brevets

(11) **EP 4 682 788 A2**

(12) **EUROPEAN PATENT APPLICATION**

(43) Date of publication:
**21.01.2026   Bulletin 2026/04**

(21) Application number: **25189253.5**

(22) Date of filing: **14.07.2025**

(51) International Patent Classification (IPC):
**G06Q 10/04** (2023.01)     **G01N 33/00** (2006.01)
**G06Q 10/0635** (2023.01)     **G06Q 50/26** (2024.01)

(52) Cooperative Patent Classification (CPC):
**G06Q 10/04; G06Q 10/0635; G06Q 50/26**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **16.07.2024   KR 20240093709**

(71) Applicant: **Korea Institute of Ocean Science and Technology**
**Busan 49111 (KR)**

(72) Inventors:
• **LEE, Moonjin**
**34033 Daejeon (KR)**
• **KIM, Tae Sung**
**35209 Daejeon (KR)**
• **KIM, Yong Myung**
**34829 Daejeon (KR)**

(74) Representative: **EP&C**
**P.O. Box 3241**
**2280 GE Rijswijk (NL)**

(54)   **METHOD FOR ESTIMATING DIFFUSION CHARACTERISTICS OF MARINE HAZARDOUS AND NOXIOUS SUBSTANCES USING SUBSTITUTES**

(57)   The present invention relates to a system and method for accurately estimating the dispersion characteristics of hazardous and noxious substances (HNS) released into the marine environment. According to the invention, HNS are classified into 12 groups based on their physical and chemical properties. For each of the three behavioral characteristics-floating, dissolution, and sinking-substitutes are used to observe and analyze the dispersion behavior of HNS in the ocean. Based on the analysis results, the system enables the estimation of floating, dissolution, and sinking behavior characteristics without the need to release actual HNS into the sea. Thus, a marine HNS dispersion characteristics estimation system and method using substitutes is provided.

【Figure 2】

**Description**

**CROSS-REFERENCE TO RELATED APPLICATIONS**

This application claims priority to Korean Patent Application No. 10-2024-0093709 filed on July 16, 2024, the entire contents of which are herein incorporated by reference.

**TECHNICAL FIELD**

The present invention relates to a method for understanding the diffusion characteristics of various hazardous and noxious substances (HNS) spilled at sea. More specifically, in order to assess the environmental impact of HNS spills or discharges in the marine environment, it is essential to accurately determine the diffusion characteristics of HNS in seawater through simulation. However, conventional methods for estimating HNS diffusion characteristics have been limited in achieving accurate results because, while it is necessary to release HNS into the actual sea and observe the diffusion process, doing so is impossible due to the hazardous nature of HNS. To overcome this problem, the present invention provides a method for estimating the diffusion characteristics of hazardous and noxious substances at sea using a simulant, wherein the diffusion characteristics of HNS are determined by employing a simulant in place of actual HNS release into the marine environment.

Furthermore, the present invention aims to resolve the problems of conventional HNS diffusion characteristic estimation methods, which have been limited in accurately determining diffusion characteristics due to the impossibility of releasing HNS into the actual marine environment for measurement. To this end, HNS are classified into 12 groups according to their physicochemical properties, and the diffusion characteristics of HNS are observed and analyzed using simulants for each of the three behavioral characteristics: floating, dissolution, and sinking. Based on the results of these observations and analyses, the method calculates the floating, dissolution, and sinking behavioral characteristics of HNS in the marine environment. Thus, the present invention provides a method for estimating the diffusion characteristics of hazardous and noxious substances at sea using a simulant. This method allows for a more accurate determination of HNS diffusion characteristics without the need to release actual HNS into the sea for measurement.

**BACKGROUND**

Recently, as environmental issues have emerged as a global concern, there has been increasing interest in hazardous and noxious substances (HNS), which can be discharged or spilled into the sea and have various adverse effects on the marine environment.

As environmental issues have emerged as a global concern, interest in hazardous and noxious substances (HNS), which can be discharged or spilled into the sea and have various adverse impacts on the marine environment, has significantly increased.

For example, as disclosed in Korean Patent Registration No. 10-1740720, "Apparatus and Method for Predicting Diffusion of Hazardous and Noxious Substances," and Korean Patent Registration No. 10-1751020, "Method and Apparatus for Continuous Detection of Hazardous and Noxious Substances Based on Multiple Satellites," various technologies have been proposed to enable rapid and effective response by predicting the risk radius of HNS diffusion in the marine environment, detecting HNS, and determining their flow paths. However, such conventional technologies have the following limitations.

More specifically, according to the current Marine Environment Management Act, a total of 799 types of hazardous and noxious substances (HNS) are designated. In order to assess the impact of HNS spills or discharges on the marine environment, it is essential to simulate and predict the diffusion of HNS in the sea, which first requires accurate determination of the diffusion characteristics of HNS in the marine environment.

To accurately determine the diffusion characteristics of HNS in the sea, releasing HNS into the actual marine environment and observing the diffusion process would yield the most precise results. However, due to the hazardous nature of HNS, releasing them into the sea is prohibited by current laws.

To address this problem, it is necessary to obtain results equivalent or similar to actual measurements without releasing HNS into the sea. However, the conventional technologies described above do not provide such technical solutions, which is another limitation.

Therefore, to overcome the limitations of conventional technologies, it is desirable to propose a new method for estimating the diffusion characteristics of hazardous and noxious substances at sea using a simulant, which is designed to more accurately determine the diffusion characteristics of HNS by using a simulant instead of releasing HNS into the sea. However, to date, no device or method that fully satisfies these requirements has been presented.

## SUMMARY

The present invention is intended to resolve the problems of the conventional technologies described above. Accordingly, the purpose of the present invention is to provide a method for estimating the diffusion characteristics of hazardous and noxious substances at sea using a simulant. In order to assess the environmental impact of HNS spills or discharges in the marine environment, it is essential to accurately determine the diffusion characteristics of HNS in the sea through simulation. To achieve this, it is necessary to release HNS into the actual sea and observe the diffusion process; however, due to the hazardous nature of HNS, releasing them into the sea is impossible, making it difficult to accurately determine their diffusion characteristics. The present invention overcomes this limitation by using a simulant instead of actual HNS to assess the diffusion characteristics of HNS in the marine environment.

Furthermore, another purpose of the present invention is to resolve the problems of conventional HNS diffusion characteristic estimation methods, which have been limited in accurately determining diffusion characteristics due to the impossibility of releasing HNS into the actual sea for measurement. To this end, HNS are classified into 12 groups based on their physicochemical properties, and the diffusion characteristics of HNS are observed and analyzed using simulants for each of the three behavioral characteristics: floating, dissolution, and sinking. Based on the results of these observations and analyses, the method calculates the floating, dissolution, and sinking behavioral characteristics of HNS in the marine environment. Thus, the present invention provides a method for estimating the diffusion characteristics of hazardous and noxious substances at sea using a simulant, enabling a more accurate determination of HNS diffusion characteristics without the need to release actual HNS into the sea for measurement.

To achieve the above objectives, the present invention provides a method for estimating the dispersion characteristics of hazardous and noxious substances (HNS) in the marine environment, the method comprising: a data collection step, in which various predetermined data are collected for the purpose of estimating the dispersion characteristics of hazardous and noxious substances (HNS) in the marine environment; a floating behavior characteristic calculation step, in which the floating behavior characteristics of the HNS are calculated based on the data collected in the data collection step; a dissolving behavior characteristic calculation step, in which the dissolving behavior characteristics of the HNS are calculated based on the data collected in the data collection step; and a sinking behavior characteristic calculation step, in which the sinking behavior characteristics of the HNS are calculated based on the data collected in the data collection step; wherein all of the above processes are configured to be executed by a computer or dedicated hardware.

In addition, in an embodiment the method further comprises: an output step, in which the data obtained from the processing procedures and results of each step-including the calculated behavior characteristics-are output in a specific form or format, based on user requests or predefined settings; a database construction step, in which the data obtained from the processing procedures and results of each step-including the calculated behavior characteristics-are stored in a database according to predefined settings, thereby constructing a database related to the behavior characteristics of HNS; and a data provision step, in which the data obtained from the processing procedures and results of each step-including the calculated behavior characteristics-are transmitted to an external device, including a user terminal, in response to user requests, thereby providing customized information services related to the behavior characteristics of HNS.

Additionally, in an embodiment the data collection step is configured such that HNS simulants are drifted on the ocean surface, their movement trajectories are tracked, and various measured values related to the drift and dispersion characteristics of HNS- including travel time, travel distance, travel speed, travel direction, time-dependent dispersion area, and variations in dispersion range-are obtained either by direct input or by receiving the data from external sources through simulation of HNS dispersion characteristics.

Furthermore, in an embodiment in the floating behavior characteristic calculation step, the drift characteristics of HNS, including drift speed and direction, are calculated by tracking the movement trajectories of HNS simulants and using the measured travel time and travel distance according to the following equation:

$$\text{Drift speed} = \text{Travel distance} / \text{Travel time}$$

Using the following equation, the horizontal dispersion characteristics of HNS are calculated by determining the dispersion area based on the movement trajectories of multiple simulants released from the same starting point:

$$\sigma^2(t) = \frac{2}{N(N-1)} \sum_{i=1}^{N} \sum_{i \neq j}^{N} [(x_i(t) - x_j(t))^2 + (y_i(t) - y_j(t))^2]$$

(where, N is the number of simulants, and xi and yi denote the coordinates of the i-th simulant's trajectory).

Based on the calculated drift characteristics and horizontal dispersion characteristics, the floating behavior characteristics of HNS are estimated.

Moreover, in an embodiment in the dissolving behavior characteristic calculation step, the drift characteristics of HNS, including drift speed and direction, are calculated by tracking the movement trajectories of HNS simulants and using the measured travel time and travel distance according to the following equation:

$$\text{Drift speed} = \text{Travel distance} / \text{Travel time}$$

Using the following equation, the horizontal dispersion characteristics of HNS are calculated by determining the dispersion area from the movement trajectories of multiple simulants released from the same starting point:

$$\sigma^2(t) = \frac{2}{N(N-1)} \sum_{i=1}^{N} \sum_{i \neq j}^{N} [(x_i(t) - x_j(t))^2 + (y_i(t) - y_j(t))^2]$$

(where, N is the number of simulants, and xi and yi denote the coordinates of the i-th simulant's trajectory).

Furthermore, by deploying two or more underwater HNS simulants at two or more depths and tracking their movement trajectories, the vertical behavior characteristics of HNS are calculated based on the measured vertical velocity, using the following equation:

$$\omega = -\delta z \left( \frac{\partial u}{\partial x} + \frac{\partial v}{\partial y} \right)$$

(where, $\omega$ is the vertical velocity, $\delta z$ is the depth difference between simulants, and $u$ and $v$ are the current velocities in the x and y directions, respectively).

Additionally, the vertical mixing characteristics of HNS are calculated by deploying underwater HNS simulants at two or more depths (z1, z2) and tracking their movement velocities, using the following equation:

$$Uz_2 = Uz_1 \exp(-k\delta z)$$

$$k = \frac{\ln Uz_1 - \ln Uz_2}{\delta z}$$

(where, $k$ is the vertical diffusion coefficient, $Uz1$ and $Uz2$ are the movement velocities at depths $z1$ and $z2$, respectively, and $\delta z$ is the depth difference).

Based on the calculated drift characteristics, horizontal dispersion characteristics, vertical behavior characteristics, and vertical mixing characteristics, the dissolving behavior characteristics of HNS are estimated.

Additionally, in an embodiment in the sinking behavior characteristic calculation step, two or more underwater HNS simulants are deployed at two or more depths, with at least two simulants at each depth, and their movement trajectories are tracked. The vertical behavior characteristics of the HNS are calculated from the measured movement velocities using the following equation:

$$\omega = -\delta z \left( \frac{\partial u}{\partial x} + \frac{\partial v}{\partial y} \right)$$

(where, $\omega$ is the vertical velocity, $\delta z$ is the depth difference between the simulants, and $u$ and $v$ represent the current velocities in the x and y directions, respectively).

Based on the calculated vertical behavior characteristics of the HNS, the sinking behavior characteristics are estimated.

According to the present invention, a marine hazardous and noxious substance (HNS) diffusion characteristic estimation system comprises: a data collection unit configured to perform a process of collecting various predetermined data for estimating the diffusion characteristics of the marine hazardous and noxious substances (HNS); a behavior characteristic estimation unit configured to estimate the behavior characteristics of the HNS based on the data collected by the data collection unit; an output unit configured to perform a process of outputting various data obtained from each processing step and result according to predetermined settings; a database unit configured to store various data obtained from each processing step and result to build a database related to the behavior characteristics of the HNS; a communication unit configured to perform processes of transmitting and receiving various data by communicating with external devices through at least one of wireless or wired communication methods; and a control unit configured to control the overall operation of the system. The behavior characteristic estimation unit is configured to perform processes of estimating the floating, dissolving, and sinking behavior characteristics of the marine hazardous and noxious substances (HNS) using the marine hazardous and noxious substance diffusion characteristic estimation method described above. Thus, the marine hazardous and noxious substance (HNS) diffusion characteristic estimation system is provided.

Moreover, according to the present invention, a marine hazardous and noxious substance (HNS) information service providing system comprises: user terminals through which individual users request and receive desired services; and a service server configured to provide corresponding services according to the requests received from the respective user terminals. The service server estimates the floating, dissolving, and sinking behavior characteristics of the marine hazardous and noxious substances (HNS) using the marine hazardous and noxious substance diffusion characteristic estimation method described above, and provides various information related to the behavior characteristics of the marine hazardous and noxious substances (HNS) in a customized manner to each user's terminal according to the user's request. Thus, the marine hazardous and noxious substance (HNS) information service providing system is provided.

Here, the user terminal is characterized in that it is configured by installing a dedicated application that is linked to the service server on a personal mobile information and communication device, such as a smartphone or tablet PC, or on an information processing device including a PC or laptop.

As described above, according to the present invention, a method for estimating the diffusion characteristics of marine hazardous and noxious substances (HNS) using simulants is provided, wherein the HNS are classified into 12 groups based on their physicochemical properties, and the diffusion characteristics of the HNS are observed and analyzed using simulants for each of the three behavior characteristics: floating, dissolving, and sinking. Based on these observations, processes for calculating the floating behavior characteristics, dissolving behavior characteristics, and sinking behavior characteristics of the HNS in the marine environment are performed. Consequently, it is possible to more accurately understand the diffusion characteristics of HNS without dispersing HNS directly into the actual marine environment for measurement.

Furthermore, according to the present invention, by providing the above-mentioned method using simulants to more accurately grasp the diffusion characteristics of HNS, it addresses the problems of conventional HNS diffusion characteristic estimation methods, where precise identification of diffusion characteristics was difficult due to the impossibility of dispersing HNS into the actual marine environment because of their hazardous nature. This is especially important as simulating the diffusion of HNS in the marine environment and accurately understanding their diffusion characteristics are essential for environmental impact assessments related to marine spills or discharges of HNS.

**BRIEF DESCRIPTION OF THE DRAWINGS**

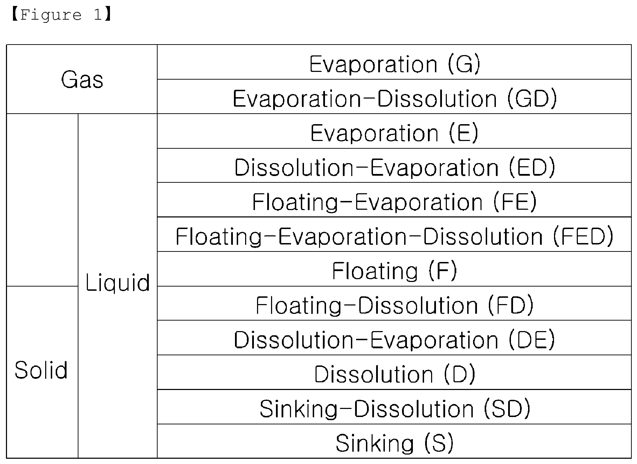

Figure 1 is a diagram illustrating an example of HNS classification applied to the method for estimating marine hazardous and noxious substance diffusion characteristics using simulants according to an embodiment of the present invention, summarized in a table.

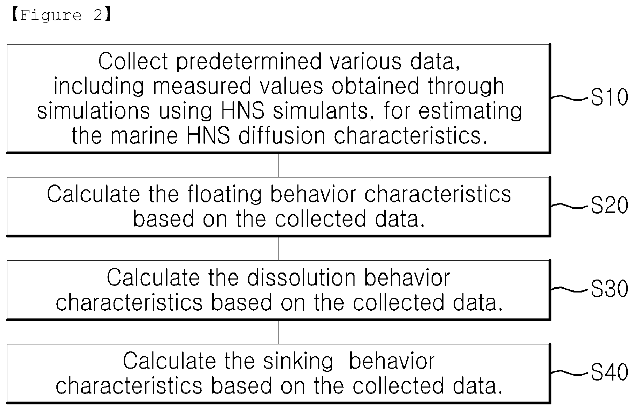

Figure 2 is a flowchart schematically showing the overall configuration of the method for estimating marine hazardous and noxious substance diffusion characteristics using simulants according to an embodiment of the present invention.

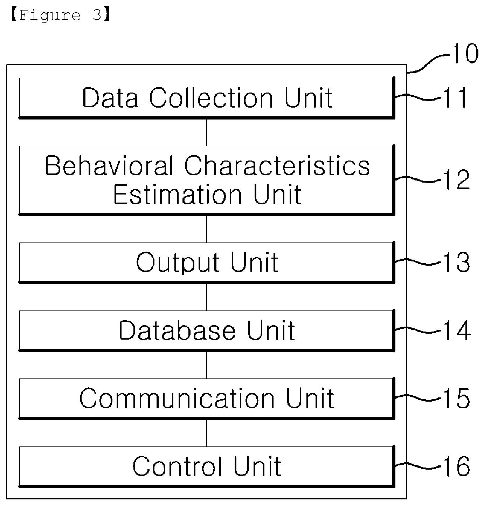

Figure 3, with reference to Figures 1 and 2, is a block diagram schematically illustrating the overall configuration of a marine hazardous and noxious substance (HNS) diffusion characteristic estimation system that estimates the behavior characteristics of HNS using the method for estimating marine hazardous and noxious substance diffusion characteristics using simulants according to the embodiment of the present invention.

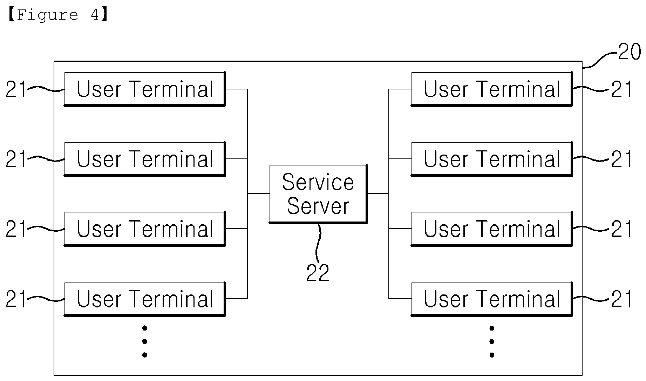

Figure 4, with reference to Figures 1 and 2, is a block diagram schematically illustrating the overall configuration of a marine hazardous and noxious substance (HNS) information service providing system that provides various information related to the behavior characteristics of HNS customized according to user requests by using the method for estimating marine hazardous and noxious substance diffusion characteristics using simulants according to the embodiment of the present invention.

## DETAILED DESCRIPTION OF THE PREFERRED EMBODIMENTS

Hereinafter, with reference to the accompanying drawings, a specific embodiment of the method for estimating marine hazardous and noxious substance (HNS) diffusion characteristics using simulants according to the present invention will be described in detail.

It should be noted that the following description is merely one exemplary embodiment for implementing the present invention, and the present invention is not limited to the content of the embodiment described below.

Further, in the description of the embodiments of the present invention below, detailed explanations are omitted for portions that are identical or similar to the prior art or that can be easily understood and implemented by those skilled in the art, for the sake of brevity.

Continuing with reference to the drawings, the specific details of the method for estimating marine hazardous and noxious substance diffusion characteristics using simulants according to the present invention will be described.

More specifically, conventionally, in order to accurately assess the impact of various hazardous and noxious substances (HNS) spilled into the marine environment, it is essential to precisely understand the diffusion characteristics of HNS in the ocean. However, since it is not possible to simulate the diffusion process by directly releasing HNS into the actual marine environment, there has been a limitation in accurately grasping the diffusion characteristics based on actual observations.

To address the above-mentioned problem, the present invention proposes a method for estimating marine hazardous and noxious substance diffusion characteristics using simulants, which enables the diffusion characteristics of HNS to be identified in a manner similar to reality by using simulants instead of releasing HNS into the ocean.

For this purpose, as described below, the present invention classifies HNS into twelve groups based on their physicochemical properties, and observes and analyzes the diffusion characteristics of HNS for three behavioral types-floating, dissolving, and sinking-by using corresponding simulants. Based on such analysis results, the method calculates the drifting and horizontal diffusion characteristics on the ocean surface, as well as vertical behavior and vertical mixing characteristics, and combines these results to estimate the floating, dissolving, and sinking behavior characteristics of HNS. Through this process, the diffusion characteristics of HNS can be accurately determined without releasing HNS into the actual marine environment.

More specifically, referring to FIG. 1, FIG. 1 is a diagram illustrating an example of the classification of hazardous and noxious substances (HNS) applied in the method for estimating the diffusion characteristics of marine hazardous and noxious substances using simulants according to an embodiment of the present invention, summarized in a table.

As shown in FIG. 1, in the present invention, HNS are classified according to their physicochemical properties. For gaseous HNS, the classification includes evaporation (G) and evaporative dissolution (GD). For liquid HNS, the classification includes evaporation (E), dissolution-evaporation (ED), floating evaporation (FE), floating evaporation-dissolution (FED), floating (F), floating dissolution (FD), dissolution-evaporation (DE), dissolution (D), sinking dissolution (SD), and sinking (S). For solid HNS, the classifications include floating dissolution (FD), dissolution-evaporation (DE), dissolution (D), sinking dissolution (SD), and sinking (S). Among these twelve classified groups of HNS behavior characteristics, the present invention proposes a method to observe and analyze the three behavior characteristics-floating, dissolving, and sinking-using simulants.

That is, first, regarding the floating behavior characteristics, in order to estimate the floating behavior characteristics of HNS, it is necessary to understand the drifting characteristics and horizontal diffusion characteristics on the ocean surface. To this end, in the present invention, a vinyl-sheet type surface-floating HNS simulant is allowed to drift on the ocean surface, and its movement path is tracked to determine drifting characteristics such as drift speed and direction based on the measured travel time and travel distance.

Here, the drift speed can be calculated using the following [Equation 1].

[Equation 1]

$$\text{Drift velocity} = \text{Travel distance} / \text{Travel time}$$

Regarding the horizontal diffusion characteristics, three or more floating HNS simulants are drifted on the sea surface, and their trajectories are tracked. By analyzing the positions of each simulant at the same time points, diffusion characteristics such as the diffusion area and the variation of the diffusion range over time are determined.

Furthermore, the diffusion area from the multiple simulants departing from the same point can be calculated as the variance of the distances between the simulants, as shown in the following equation:

[Equation 2]

$$\sigma^2(t) = \frac{2}{N(N-1)} \sum_{i=1}^{N} \sum_{i \neq j}^{N} [(x_i(t) - x_j(t))^2 + (y_i(t) - y_j(t))^2]$$

Here, in Equation 2, N denotes the number of simulants, and xi and yi represent the coordinates of the trajectory of the i-th simulant, respectively.

Next, regarding the dissolution behavior characteristics, it is necessary to understand the drifting characteristics, horizontal diffusion characteristics, vertical behavior characteristics, and vertical mixing characteristics underwater. To this end, for the drifting characteristics and horizontal diffusion characteristics underwater, underwater HNS simulants are deployed at the target analysis depth, and their trajectories are tracked. The drifting and horizontal diffusion characteristics underwater can be determined by applying the same method as used for calculating the drifting and horizontal diffusion characteristics on the sea surface.

Further, the vertical behavior characteristics underwater are determined by deploying at least two underwater HNS simulants at two or more depths, respectively, tracking their trajectories, and calculating the vertical velocity using the continuity equation as shown in Equation 3 below, based on the measured velocities.

[Equation 3]

$$\omega = -\delta z \left( \frac{\partial u}{\partial x} + \frac{\partial v}{\partial y} \right)$$

In Equation 3, $\omega$ denotes the vertical velocity, $\delta z$ represents the difference in depth between the simulants, and $u$ and $v$ indicate the flow velocities in the $x$- and $y$-directions, respectively.

Furthermore, the vertical mixing characteristics underwater are determined by deploying underwater HNS simulants at two or more depths (z1, z2), tracking their trajectories, and calculating based on the measured velocities as shown in Equation 4 below.

[Equation 4]

$$Uz_2 = Uz_1 \exp(-k\delta z)$$

$$k = \frac{\ln Uz_1 - \ln Uz_2}{\delta z}$$

In Equation 4, k denotes the vertical diffusion coefficient, Uz1 and Uz2 represent the velocities at depths z1 and z2, respectively, and $\delta z$ is the difference between z1 and z2.

Next, regarding the sinking behavior characteristics, it is necessary to understand the vertical behavior characteristics underwater. Since the vertical behavior characteristics underwater can be applied from the characteristics identified in the dissolution behavior, detailed explanation of overlapping content is omitted herein.

Furthermore, as described above, the process of drifting HNS simulants on the sea surface and tracking their trajectories to calculate the floating, dissolution, and sinking behavior characteristics of HNS can be implemented by those skilled in the art with reference to conventional technologies such as known marine floating body trajectory tracking methods. Therefore, for brevity, detailed descriptions of content that are apparent to those skilled in the art or can be readily understood and implemented by referring to conventional literature are omitted herein.

Accordingly, from the foregoing, the HNS diffusion characteristic estimation method using simulants according to the embodiment of the present invention can be easily implemented. Referring to FIG. 2, FIG. 2 schematically illustrates the overall configuration of the HNS diffusion characteristic estimation method using simulants according to the present embodiment.

As shown in FIG. 2, the HNS diffusion characteristic estimation method using simulants according to the embodiment of the present invention broadly includes: a data collection step (S10) in which various predetermined data are collected for estimating the diffusion characteristics of hazardous and noxious substances (HNS), including measured values obtained by drifting HNS simulants on the sea surface and tracking their trajectories through simulation of HNS diffusion characteristics; a floating behavior characteristic calculation step (S20) in which floating behavior characteristics are calculated based on the data collected in the data collection step (S10); a dissolution behavior characteristic calculation step (S30) in which dissolution behavior characteristics are calculated based on the data collected in the data collection step (S10); and a sinking behavior characteristic calculation step (S40) in which sinking behavior characteristics are calculated based on the data collected in the data collection step (S10). These processing steps may be performed by a computer or dedicated hardware.

Here, it should be noted that the respective calculation steps (S20, S30, S40) are not necessarily performed sequentially in the order shown in FIG. 2, but may be carried out in any order. Furthermore, the calculation steps may be performed in parallel or simultaneously, and may be configured flexibly according to needs.

Further, although not explicitly illustrated, the aforementioned method may further include: an output step in which each piece of data obtained from the processing steps and results-including the calculated behavior characteristic results-is output in a specific form or format according to a user's request or predetermined settings; a database construction step in which each piece of data obtained from the processing steps and results-including the calculated behavior characteristic results-is stored in a database according to predetermined settings, thereby constructing a database related to the behavior characteristics of HNS; and a data provision step in which the data obtained from the processing steps and results-including the calculated behavior characteristic results-is transmitted to external devices such as user terminals according to the user's request, thereby providing a user-customized information service related to the behavior characteristics of HNS.

More specifically, the aforementioned data collection step (S10) may be configured to perform a process of directly inputting or receiving from an external source various simulation measurement values related to the drifting characteristics and diffusion characteristics of HNS, such as travel time, travel distance, travel speed, travel direction, time-dependent diffusion area, and diffusion range variation, obtained through the HNS behavior characteristic simulation using the aforementioned simulant.

Meanwhile, the aforementioned floating behavior characteristic calculation step (S20), dissolution behavior characteristic calculation step (S30), and sinking behavior characteristic calculation step (S40) may be configured to perform processes of calculating each behavior characteristic as described above with reference to the aforementioned [Mathematical Equations 1] to [4].

Herein, although the present invention has been described by way of an example where the floating, dissolution, and sinking behavior characteristics of HNS are calculated based on data collected through simulations using HNS simulants, the present invention is not limited only to the contents presented in the above-described embodiment. That is, the present invention may be configured to estimate various behavior characteristics in addition to the configurations presented in the embodiment. It should be noted that the present invention may be modified and changed in various ways by those skilled in the art within the scope without departing from the spirit and essential features of the invention.

Accordingly, as described above, the HNS diffusion characteristic estimation method using simulants according to the embodiment of the present invention can be implemented. By utilizing this, it is possible to easily implement an HNS diffusion characteristic estimation system and a user-customized information service that provides various information related to the behavior characteristics of hazardous and noxious substances (HNS) according to user requests.

Referring to FIG. 3, FIG. 3 is a block diagram schematically illustrating the overall configuration of the hazardous and noxious substance (HNS) diffusion characteristic estimation system 10 that estimates the behavior characteristics of HNS by using the HNS diffusion characteristic estimation method using simulants according to the embodiment of the present invention, with reference to FIGS. 1 and 2.

As shown in FIG. 3, the hazardous and noxious substance (HNS) diffusion characteristic estimation system 10 according to the embodiment of the present invention includes a data collection unit 11 configured to perform processing for collecting various predetermined data for estimating the diffusion characteristics of HNS; a behavior characteristic estimation unit 12 configured to estimate the behavior characteristics of HNS based on the data collected through the data collection unit 11; an output unit 13 configured to perform processing for outputting the data obtained from each processing step and result according to a predetermined setting; a database unit 14 configured to perform processing for storing various data obtained from each processing step and result to construct a database related to the behavior characteristics of HNS; a communication unit 15 configured to perform processing for transmitting and receiving various data through at least one of wireless or wired communication with external devices; and a control unit 16 configured to perform processing for controlling the overall operation of each unit and the system 10.

Here, the behavior characteristic estimation unit 12 is configured to perform processing for estimating the floating, dissolving, and sinking behavior characteristics of hazardous and noxious substances (HNS) using the HNS diffusion characteristic estimation method based on simulants according to the embodiment of the present invention, with reference to FIGS. 1 and 2.

Further, referring to FIG. 4, FIG. 4 is a block diagram schematically illustrating the overall configuration of the hazardous and noxious substance (HNS) information service providing system 20 that provides various information related to the behavior characteristics of HNS in a customized manner according to user requests by using the HNS diffusion characteristic estimation method based on simulants according to the embodiment of the present invention, with reference to FIGS. 1 and 2.

As shown in FIG. 4, the hazardous and noxious substance (HNS) information service providing system 20 according to the embodiment of the present invention includes a user terminal 21, by which each user requests and receives desired services, and a service server 22 configured to provide corresponding services according to user requests received through each user terminal 21.

Here, the user terminal 21 may be configured as dedicated hardware linked with the service server 22; however, preferably, it may be configured by installing a dedicated application linked with the service server 22 on portable information communication devices such as smartphones or tablet PCs, or information processing devices such as PCs or laptops.

Meanwhile, the service server 22 may be configured to estimate the floating, dissolving, and sinking behavior characteristics of hazardous and noxious substances (HNS) by using the HNS diffusion characteristic estimation method based on simulants according to the embodiment of the present invention with reference to FIGS. 1 and 2, and to provide the corresponding information to the user's user terminal 21 upon request.

Accordingly, the present invention can implement the HNS diffusion characteristic estimation method based on simulants according to the embodiment described above, whereby HNS is classified into twelve groups based on physicochemical properties, and for the three behavior characteristics of floating, dissolving, and sinking, the diffusion characteristics of HNS are observed and analyzed by using simulants. Based on these results, floating, dissolving, and sinking behavior characteristics of HNS in the marine environment are calculated. This allows relatively accurate identification of the diffusion characteristics of HNS without actually releasing HNS into the marine environment for measurement. Consequently, the present invention overcomes the problems of conventional methods for estimating HNS diffusion characteristics, which had difficulty in accurate identification due to the impossibility of releasing hazardous HNS into the marine environment because of its risk.

The above-described embodiments provide a detailed description of the HNS diffusion characteristic estimation method using simulants according to the present invention. However, the present invention is not limited to the embodiments described above, and various modifications, changes, combinations, and substitutions may be made by those skilled in the art according to design requirements and other various factors within the scope of the technical spirit of the present invention.

## Claims

1.   A method for estimating the dispersion characteristics of hazardous and noxious substances (HNS) in the marine environment, the method comprising:

a data collection step in which various predetermined data necessary for estimating the dispersion characteristics of HNS are collected;
a floating behavior characteristic estimation step in which the floating behavior characteristics of HNS are calculated based on the data collected in the data collection step;
a dissolution behavior characteristic estimation step in which the dissolution behavior characteristics of HNS are calculated based on the data collected in the data collection step; and
a sinking behavior characteristic estimation step in which the sinking behavior characteristics of HNS are calculated based on the data collected in the data collection step;
wherein the respective processes are performed by a computer or dedicated hardware.

2.   The method according to claim 1,
wherein the method further comprises:

an output step in which each of the data obtained through the processing and results of each step, including the estimated behavior characteristics, is output in a specific form or format according to a user's request or a predetermined setting;
a database construction step in which each of the data obtained through the processing and results of each step, including the estimated behavior characteristics, is stored in a database according to a predetermined setting to construct a database related to the behavior characteristics of HNS; and
a data provision step in which each of the data obtained through the processing and results of each step, including the estimated behavior characteristics, is transmitted to an external device including a user terminal in response to a user's request to provide user-customized information services related to HNS behavior characteristics.

3. The method according to claim 1 or claim 2,
   wherein the data collection step is configured to perform a process of directly receiving or receiving from an external source various measured values related to the drift and diffusion characteristics of HNS, including drift time, travel distance, travel speed, travel direction, time-based diffusion area, and diffusion range variation, which are measured through a simulation of HNS diffusion characteristics by drifting an HNS simulant on the sea surface and tracking its movement path.

4. The method according to any one of the preceding claims,
   wherein the floating behavior characteristic calculation step is configured to:

   calculate the drift characteristics of HNS, including drift speed and direction, by tracking the movement path of the HNS simulant and applying the following equation using the measured travel time and travel distance:

$$\text{Drift speed} = \text{Travel distance} / \text{Travel time}$$

   and calculate the horizontal diffusion characteristics of HNS by computing the diffusion area from the movement paths of multiple simulants released from the same starting point using the following equation:

$$\sigma^2(t) = \frac{2}{N(N-1)} \sum_{i=1}^{N} \sum_{i \neq j}^{N} [(x_i(t) - x_j(t))^2 + (y_i(t) - y_j(t))^2]$$

   (where N is the number of simulants, and xi and yi represent the movement coordinates of the i-th simulant), and estimate the floating behavior characteristics of HNS based on the derived drift and horizontal diffusion characteristics.

5. The method according to any one of the preceding claims,
   wherein the dissolution behavior estimation step comprises:

   Calculating the drift characteristics of HNS, including drift speed and direction, by tracking the movement path of HNS simulants and using the measured travel time and distance according to the following equation:

$$\text{Drift speed} = \text{Travel distance} / \text{Travel time}$$

   Calculating the horizontal diffusion characteristics of HNS using the movement paths of multiple simulants launched from the same point, based on the following equation:

$$\sigma^2(t) = \frac{2}{N(N-1)} \sum_{i=1}^{N} \sum_{i \neq j}^{N} [(x_i(t) - x_j(t))^2 + (y_i(t) - y_j(t))^2]$$

   (where N is the number of simulants, and xi and yi represent the coordinates of the i-th simulant)
   Calculating the vertical behavior characteristics of HNS using the following equation by deploying two or more underwater HNS simulants at two or more depths, tracking their movement paths, and using the measured movement speed:

$$\omega = -\delta z \left( \frac{\partial u}{\partial x} + \frac{\partial v}{\partial y} \right)$$

   (where $\omega$ is the vertical velocity, $\delta z$ is the depth difference between simulants, and u and v are the current velocities in the x and y directions, respectively)

Calculating the vertical mixing characteristics of HNS using the following equation by deploying underwater HNS simulants at two or more depths (z1, z2), tracking their movement paths, and using the measured movement speed:

$$Uz_2 = Uz_1 \exp\left(-k\delta z\right)$$

$$k = \frac{\ln Uz_1 - \ln Uz_2}{\delta z}$$

(where k is the vertical diffusion coefficient, Uz1 and Uz2 are the movement speeds at depths z1 and z2, and $\delta z$ is the difference between z1 and z2)

And estimating the dissolution behavior of HNS based on the derived drift characteristics, horizontal diffusion characteristics, vertical behavior characteristics, and vertical mixing characteristics.

6. The method according to any one of the preceding claims,
wherein the sinking behavior estimation step comprises:

Calculating the vertical behavior characteristics of HNS by deploying two or more underwater HNS simulants at two or more depths, tracking their movement paths, and measuring their movement speeds using the following equation:

$$\omega = -\delta z \left( \frac{\partial u}{\partial x} + \frac{\partial v}{\partial y} \right)$$

(where $\omega$ is the vertical velocity, $\delta z$ is the depth difference between the simulants, and u and v represent the current velocities in the x and y directions, respectively) Estimating the sinking behavior characteristics based on the calculated vertical behavior characteristics of HNS.

7. A hazardous and noxious substance (HNS) diffusion characteristic estimation system, comprising:

a data collection unit configured to perform a process of collecting various predetermined data for estimating the diffusion characteristics of HNS;
a behavior characteristic estimation unit configured to perform a process of estimating the behavior characteristics of HNS based on the data collected through the data collection unit;
an output unit configured to perform a process of outputting data obtained from each process and result according to predetermined settings;
a database unit configured to perform a process of storing various data obtained from each process and result to construct a database related to the behavior characteristics of HNS;
a communication unit configured to perform processes of transmitting and receiving various data by communicating with external devices via at least one of wireless or wired communication;
and a control unit configured to perform processes of controlling the overall operation of the system,
wherein the behavior characteristic estimation unit is configured to perform a process of estimating the floating, dissolution, and sinking behavior characteristics of HNS respectively, by using the hazardous and noxious substance diffusion characteristic estimation method according to claim 1.

8. A hazardous and noxious substance (HNS) information service providing system,
comprising:

a user terminal configured to request and receive desired services from respective users; and
a service server configured to provide corresponding services according to user requests received through the respective user terminals,
wherein the service server is configured to estimate the floating, dissolution, and sinking behavior characteristics of hazardous and noxious substances (HNS) using the hazardous and noxious substance diffusion characteristic estimation method according to claim 1, and

to provide various information related to the behavior characteristics of HNS in a customized manner to the user's user terminal according to the user's request.

9. The hazardous and noxious substance (HNS) information service providing system according to claim 8, wherein the user terminal is configured by installing a dedicated application linked with the service server on a personal portable information communication device including a smartphone or tablet PC, or on an information processing device including a PC or laptop.

【Figure 1】

| Gas | | Evaporation (G) |
|---|---|---|
| | | Evaporation-Dissolution (GD) |
| Solid | Liquid | Evaporation (E) |
| | | Dissolution-Evaporation (ED) |
| | | Floating-Evaporation (FE) |
| | | Floating-Evaporation-Dissolution (FED) |
| | | Floating (F) |
| | | Floating-Dissolution (FD) |
| | | Dissolution-Evaporation (DE) |
| | | Dissolution (D) |
| | | Sinking-Dissolution (SD) |
| | | Sinking (S) |

【Figure 2】

| | |
|---|---|
| Collect predetermined various data, including measured values obtained through simulations using HNS simulants, for estimating the marine HNS diffusion characteristics. | ~S10 |
| Calculate the floating behavior characteristics based on the collected data. | ~S20 |
| Calculate the dissolution behavior characteristics based on the collected data. | ~S30 |
| Calculate the sinking behavior characteristics based on the collected data. | ~S40 |

【Figure 3】

【Figure 4】

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020240093709
- KR 101740720

- KR 101751020